# EUROPEAN PATENT APPLICATION

(11) **EP 4 732 744 A1**
(43) Date of publication of application: **29.04.2026**
(21) Application number: 24208357.4
(22) Date of filing: 23.10.2024
(51) Int. Cl.: A61B 1/00, A61B 1/24, G06T 7/00

(54) **DISPLAY CHECK FOR VERIFICATION OF USER AND OUTPUT DEVICE**

(71) Applicant: DENTSPLY SIRONA Inc., York, PA 17401 (US); SIRONA Dental Systems GmbH, 64625 Bensheim (DE)
(72) Inventor: Voss, Bjoern, 64625 Bensheim (DE); Elsing, Jonas, 64625 Bensheim (DE); Kucharczyk, Ronny, 64625 Bensheim (DE)
(74) Representative: Taor, Simon Edward William

(57) **Abstract**

The invention relates to a system for displaying visual content, comprising at least one output device, and at least one controller, and at least one input device, wherein the visual content comprises features comprising a color scheme and/or at least one color group, the output device is configured to display the visual content, the controller is configured to generate a first visual test comprising at least one first test image, and to allow displaying the visual content on the output device if a user correctly identifies an object in the first test image, and to deny displaying the visual content on the output device if the user fails to correctly identify the object in the first test image, the input device is configured to receive input by the user to identify the object in the first test image. The invention is characterized in that the first test image is generated using features and/or expected features of the visual content.

## Description

### TECHNICAL FIELD OF THE INVENTION

The present invention relates to computer-implemented methods and systems for displaying visual contents.

### BACKGROUND ART OF THE INVENTION

The analysis of visual content, e.g., digital images, especially in medical, security, safety, and environmental application areas, has several requirements for both, user, and display system. On the one hand, the display system needs to be able to display the visual content correctly in terms of color fidelity, contrast, brightness, etc. On the other hand, the user must be able to interpret the displayed images correctly, i.e., must be able to distinguish grey scales, colors, and color shades in the individual use environment of the application.

For example, in medical systems that utilize screens to display images, e.g., X-ray data for diagnosis, the displays need to meet specific criteria to be approved for diagnostic purposes. In addition, the screens must be carefully calibrated upon installation and checked periodically. However, the calibration during installation does not take the user or even multiple users into account. It also may not be adapted to the use environment of the application. While the calibration might fit for one user, a second user might have a different perception and thus might misinterpret the images.

Especially for colored images, different users might perceive the shades differently. In addition to a varying perception of color shades, problems like color blindness can occur. While the user usually knows if he is prone to color blindness, especially for rather obvious cases like a heavy red-green color blindness. For more rare cases or lighter forms of color blindness, the user might not be aware that he is prone to a visual impairment.

Furthermore, sharing files, e.g., for a second opinion of a diagnosis, is very common in today's connected world. If a file, e.g., image data, is shared with someone, it's likely that the output devices are not the same and may have very different properties in terms of color display and thus the second opinion might be different only because of the display.

If a user with e.g., color blindness analyses colored pictures he might misinterpret the shown colors or color shades which can lead to a wrong diagnosis or conclusion with severe consequences for the patient or safety in general.

Also in other areas, like military, construction works, mechanical analysis, aerial observations, astronomy, quality-testing, etc. it is important that pictures are interpreted correctly. In general, for all of fluorescence, phosphorescence, absorption, luminescence, refraction, etc. need to be analyzed based on the color.

There are several methods to test color blindness. A very well-known method are test images that are composed of two colors in different shades. In these images a number, shape or object is shown in shades of one color, e.g., red, and the background is shown in shades of the other color, e.g., green. If the test person is not able to distinguish between red and green, as with red-green color blindness, he will not be able to figure out the number/shape/object shown. Usually, these color blindness test consist of a series of images with different color schemes.

While these tests can identify color blindness for users, the users can still ignore the results. Furthermore, these tests are bound to carefully calibrated screens or printed images (printed in a controlled process) to ensure that the colors and shades are displayed correctly. In everyday life, it cannot be ensured that the user does not ignore his test results or that a screen displays colors and shades in the correct way.

Some systems already provide predefined color settings for user with a known vision impairment. Using these settings solely relies on the user and the user's knowledge of possible vision impairments. If a user does not know of a vision impairment, he certainly will not use a different setting and might miss important details from the visual content. Also changes in the environment during usage, for example direct sunlight, have a clear influence on the ability of the user to assess visual information provided in an image.

### SUMMARY OF THE INVENTION

The applicant is not aware of any methods or systems in which a visual content cannot be accessed if the user cannot verify that he is able to analyze the visual content regardless of if the display is not capable of reproducing the visual content correctly or the user has any vision impairment. This is especially but not exclusively accounted to colored visual content.

The applicant is unaware of any methods or systems that would prevent the user from accessing visual content if the user is unable to verify their ability to analyze the visual content, regardless of whether the display is unable to reproduce the visual content accurately or the user has a vision impairment. This is particularly relevant, though not exclusively, in the context of colored visual content.

An objective of the present invention is to overcome at least some of the problems of the above-mentioned background art. This objective has been achieved by the system for displaying visual content as defined in claim 1, and the computer-implemented method as defined in claim 19. The dependent claims relate to further developments and preferred embodiments.

The present invention discloses a system for displaying visual content. The system comprises at least one output device, at least one controller and at least one input device. The visual content comprises features that comprise a color scheme and/or at least one color group. The visual content may include features or content that is displayed either color-coded and/or in monochrome form. The controller is configured to generate a first visual test comprising at least one first test image. The output device is configured to display the visual content as well as the first visual test. The controller is further configured to allow displaying the visual content on the display device if a user correctly identifies an object in the first test image. If the user fails to correctly identify the object in the first test image, the controller is configured to deny displaying the visual content on the output device. The input device of the system is configured to receive input by the user to identify the object in the first test image. The invention is especially characterized in that the first test image is generated using features or characteristics (color or monochrome) of the visual content. Prohibiting the display of visual content prevents wrong decisions/reactions from being made because the features or characteristics displayed cannot be perceived by the user.

The invention relates to a system for displaying visual content, comprising at least one output device, and at least one controller, and at least one input device, wherein the visual content comprises features comprising a color scheme and/or at least one color group, the output device is configured to display the visual content, the controller is configured to generate a first visual test comprising at least one first test image, and to allow displaying the visual content on the output device if a user correctly identifies an object in the first test image, and to deny displaying the visual content on the output device if the user fails to correctly identify the object in the first test image, the input device is configured to receive input by the user to identify the object in the first test image. The invention is characterized in that the first test image is generated using features and/or expected features of the visual content. Expected features are colors, color schemes (incl. monochromatic), brightness, contrast, or similar properties of the visual content that are expected to be present in the visual content based on source of the visual content and/or characteristics the visual content is to be analyzed for. The term expected features can also be used synonymously with the term features.

In some embodiments, the system is characterized in that the controller is configured to generate a modified visual test if the user fails to correctly identify the object in the first visual test and to allow displaying the visual content or a modified visual content on the output device if the user correctly identifies an object in the modified visual test, wherein the controller is configured to generate further modified visual tests until the user correctly identifies an object in at least one of the further modified visual tests or a maximum amount of failed modified visual tests is reached.

In some embodiments, the system is characterized in that the controller is configured to generate a modified visual content by modifying the visual content in dependency on the modified visual test if the modified visual test was completed correctly, and wherein the modifications of the visual content are in dependency of the modifications of the visual test, and wherein the controller is configured to allow displaying the modified visual content on the output device if the modified visual test was completed correctly.

In some embodiments, the system is characterized in that the modified visual test comprises at least one modified test image which differs from the test image of the preceding visual test in at least one or more from the following: the object, at least one color, at least one color group, the color scheme, brightness, and contrast.

In some embodiments, the system is characterized in that first visual test and/or the modified visual test comprises a plurality of objects that differ from each other in at least one selected from brightness, contrast, a color and/or a color group, wherein the controller generates an input prompt instructing the user to select the object which the user can identify comparatively better, and wherein the controller is configured to apply the color and/or color group and/or brightness and/or contrast of the selected object to the visual content.

In some embodiments, the system is characterized in that first visual test and/or the modified visual test comprises a plurality of test images that differ from each other in the object and at least one selected from brightness, contrast, a color, a color group, and the color scheme, wherein the controller generates an input prompt instructing the user to identify the object of the test image in which the user can identify the object comparatively better, and wherein the controller is configured to apply the color scheme and/or brightness and/or contrast of the selected test image to the visual content.

In some embodiments, the system is characterized in that the controller is configured to generate the plurality of test images and/or objects in dependence on the completion of the first visual test.

In some embodiments, the system is characterized in that the controller is configured to generate choice options after a visual test is failed, wherein the choice options comprise at least two options from the following:
a) generating a subsequent modified visual test, wherein an object of the test image is modified, and wherein the color scheme, and brightness, and contrast of the preceding verification task are repeated, and
b) generating a subsequent modified visual test, wherein the object in the test image as well as the color scheme are modified compared to the preceding visual test, and
c) cancel, wherein the controller is configured to deny displaying the visual content and/or not to generate modified visual content.

In some embodiments, the system is characterized in that the controller is configured to generate, together with visual test and/or after the visual test is failed, a query to retrieve information from the user if and/or why the user is not able to correctly identify the object in the test image, wherein the query comprises at least two choice options selected from contrast, brightness, color scheme, color, and color group, and wherein the controller is configured to generate a modified visual test based on the information retrieved from the user.

In some embodiments, the system is characterized in that the controller is configured to generate a new visual test after a time interval and/or at specified moments and/or before every access to a different visual content is requested by the user.

In some embodiments, the system is characterized in that controller is configured to request a validation before an output device is used to display visual content for the first time in the system, wherein the validation comprises at least one visual test and the controller is configured to allow displaying the visual content and/or modified visual content on the output device only when the respective output device is validated.

In some embodiments, the system is characterized in that the system comprises a plurality of output devices, wherein each of the plurality of output devices can be configured to display the visual content and/or modified visual content, wherein each output device is operated individually and needs to be validated before the first use in the system.

In some embodiments, the system is characterized in that when at least two output devices are used in the system, at least one output device can be excluded from the mandatory validation, wherein the controller is configured to allow displaying the visual content and/or modified visual content on the output device that is not validated, and wherein the controller is further configured to generate a notification on the output device that is not validated, that an interpretation of the visual content and/or modified visual content on the output device that is not validated is not allowed/safe.

In some embodiments, the system is characterized in that the system comprises at least one data storage, wherein the controller is configured to store user settings in the data storage, wherein the user settings are in dependency on the completion of the visual test 112 and/or the validation, and wherein the user settings comprise at least a color scheme, and wherein the user settings are automatically generated during the validation and/or visual test.

In some embodiments, the system is characterized in that the user settings are linked to individual output devices, such that if a user uses a first output device, the user settings for the first output device are loaded and then the user uses a different output device), the settings for the different output device are loaded.

In some embodiments, the system is characterized in that the system comprises at least one sensor and/or camera configured to sense at least partly the light conditions in vicinity of the output device, wherein the controller is configured to generate a visual test if the sensor and/or camera senses that the light conditions changed compared to the light conditions at a time when the preceding visual test was generated.

In some embodiments, the system is characterized in that the system is configured to analyze the colors and/or color scheme of the visual content, and the controller is configured to generate the test image and/or plurality of test images in dependence of the analysis.

In some embodiments, the system is characterized in that the controller is configured to generate the test image and/or plurality of test images in dependence of a pre-defined setting, wherein in the pre-defined setting comprises a color scheme.

The invention also related to a computing device-implemented method for displaying visual content, wherein the visual content comprises features comprising a color scheme and/or at least one color group, the method comprising the steps of
a) generating a first visual test, comprising a first test image,
b) receiving input from a user identifying an object in the first test image,
c) allowing displaying the visual content on an output device if the user correctly identifies the object in the first test image, and denying displaying the visual content on the output device if the user fails to correctly identify the object in the first test image.

The method may be characterized in that the first test image is generated using features and/or expected features of the visual content.

In some embodiments of the system, the test image comprises at least two color groups, wherein the color groups are related to the visual content and the color groups that need to be distinguishable by the user in the visual content. In some embodiments, the visual content may lack at least one of the color groups that need to be distinguishable by the user, but the test image still may comprise this missing color group, regardless of presence in the visual content. If, for example, the in the visual content red colors and green colors need to be distinguishable by the user, but the visual content only comprises red colors and lacks green colors, the visual test and/or test image may regardless comprise red colors and green colors.

In some embodiments of the system, the test image comprises an object and a background. The object is colored in at least one first color or color group, and the background is colored in at least one second color or color group that is different from the first color or color group of the object.

In some embodiments of the system, the visual test may comprise a series of different test images. The visual test is considered completed if the user attempted to identify the object correctly for each test image of the series. In some embodiments, the visual test may be considered as failed if no object in the series of test images was identified correctly. In some embodiments, the visual test may be considered completed correctly if the object is identified correctly in at least one test image of the series. In some embodiments a modified visual test is generated if the user failed to identify the object in at least one test image of the series.

In some embodiments of the system, the visual test comprises an input prompt which comprises information about how to correctly identify the object in the test image.

In some embodiments of the system, the object of the test image comprises at least one two-or three-dimensional form and/or a numeric sequence and/or an alphabetic sequence and/or an alphanumeric sequence.

In some embodiments of the system, the first visual test may comprise a plurality of test images that differ from each other in at least one selected from the following: brightness, contrast, a color, a color group, the color scheme. In these embodiments, the input prompt instructs the user to select the test image in which the user can identify the object comparatively better. Consequently, the controller is configured to apply the color scheme and/or brightness and/or contrast of the selected test image to the visual content while generating a modified visual content. If the first visual test comprises a plurality of test images, the first visual test is completed correctly if the user selects one test image of the plurality of test images.

In some embodiments of the system, a visual test comprises a plurality of test images that differ from each other in at least one selected from the following: brightness, contrast, a color, a color group, the color scheme. In some embodiments, the test images comprise a random digit and/or letter and/or an alphanumeric sequence as identifier. In these embodiments, the input prompt instructs the user to select the test image in which the user can identify the object comparatively better. Consequently, the controller is configured to apply the color scheme and/or brightness and/or contrast of the selected test image to the visual content while generating a modified visual content. If the first visual test comprises a plurality of test images, the first visual test is completed correctly if the user selects one test image of the plurality of test images by entering the respective identifier via the input device. The identifier preferably comprises the same color and/or color group as the object of the test image. In some embodiments, the identifier is the object of the test image.

In some embodiments of the system, the visual content is an image, a picture, a recorded video and/or a live feed of either pictures or video. In some embodiments, the visual content is of medical nature, e.g., a medical picture, medical image, or medical video. In some embodiments the visual content is a live feed from a scanner or camera during a medical treatment or medical procedure. In some embodiments, the visual content comprises security relevant information. In some embodiments, the visual content comprises aerial recordings. In some embodiments, the visual content comprises recordings of landscapes and/or areas, e.g., urban areas, forests, mountains, deserts, water areas, or mixtures of aforementioned areas. In some embodiments, the visual content comprises a visualization of other object or surface properties from advanced measurement techniques, such as but not limited to UV images, fluorescence images, near infrared images, spectral images or similar. In some embodiments, the visual content might comprise false color images with commonly used color representations, for example but not limited to thermal views where, for example, warm areas are marked in yellow/red colors and cold areas are marked in blue/violet colors.

In some embodiments of the system, the system is configured to analyze the visual content with respect to colors, color groups, brightness, contrast, and color scheme. In some embodiments, the system is configured to define at least two color groups for the visual content.

In some embodiments of the system, the system is configured to overlay additional information over the visual content.

In some embodiments of the system, the visual content may comprise information from x-ray measurements on material, humans or animals.

In some embodiments of the system, the system further comprises instruments and/or tools. The controller then may be configured to inhibit using these instruments and/or tools unless the first visual test and/or a modified visual test is completed correctly, i.e., the object in the test image is identified correctly. In some embodiments, the instruments may be dental instruments for a dental treatment and the visual content is related to the dental treatment.

In some embodiments of the system, the visual content comprises a tooth shade. The tooth shade may comprise any optical property of a tooth, including but not limited to color, shade, translucence, reflective properties. In case the visual content comprises a tooth shade, the visual test may be generated to test the ability of the user to distinguish certain colors on the output device. In some embodiments, the system may further comprise a tooth shade chart, e.g., a collection of objects - for example preformed teeth - in a variety of tooth shades. This tooth shade chart may be compared to objects in the visual test shown on the output device and the user is prompted to select the object which is comparatively closest to the specified tooth shade of the tooth shade chart.

In some embodiments of the system, the system comprises a data storage with pre-defined settings for the visual test and/or test image and/or plurality of test images. The pre-defined settings comprise at least an expected color scheme of the visual content, i.e., the color scheme comprising the colors and/or color groups that usually are present in the visual content to be displayed. For example, if the visual content to be displayed is a fluorescence image for caries detection, the usually present colors are green, red and greyscale colors. Thus, the test image is generated comprising at least green, red and greyscale colors.

In some embodiments of the system, the pre-defined settings further comprise information about the colors that need to be distinguished in the visual content and the test image and/or plurality of test images are generated in a way that the user needs to distinguish these colors from each other, e.g., having an object in the first color and the background in the second color. For example, if the visual content to be displayed is a fluorescence image for caries detection, usually red and green colors need to be distinguished from each other. Thus, the test image may comprise an object in green colors and a background in red colors, or the other way around.

In some embodiments, the user may input a known vision impairment, e.g., color blindness, wherein the system automatically adjusts the display settings to an adequate color scheme and/or brightness setting and/or contrast setting. In some embodiments, the system may automatically set the display settings in a way that certain colors and/or color groups are avoided for displaying the visual content. Optionally, to ensure that the automatic display settings fit for the user, a visual test is generated accordingly.

In some embodiments, the visual test may be implemented into or may be part of a software application, e.g., a software application for analyzing the visual content.

Throughout the disclosure, a color in its singular form is understood as a constant hue value on a 360-degree color wheel in terms of the HSV color space, while shade, tint, tone, brightness and/or saturation may change. For the sake of simplicity, the term color shall also include black, white and any greyscale.

Throughout the disclosure, a color group is understood as a collection of similar colors. For example, a color group can encompass a hue value range of +/- 80° around the central value, in some embodiments, the color group comprises the colors with a hue value of +/- 40° around a central value. In some embodiments, a color group may comprise the colors with a value of +/- 22° around a central value The hue value marks an angle on the color wheel with 0° being red, 120° being green, 240° being blue and 360° equals 0° and thus red. In some embodiments, color groups may partially overlap concerning their hue value. In some embodiments, the plural form of color, i.e., colors, combined with a color name may also refer to a color group. For example, red colors may refer to a color group around a hue value of 0° (pure red), e.g., +/- 22° around 0°. In the same way,
- yellow colors may refer to 60° +/- 22°, i.e., a range from 38° to 88°.
- green colors may refer to 120° +/- 22°, i.e., a range from 98° to 142°.
- cyan colors may refer to 180° +/- 22°, i.e., a range from 158° to 202°.
- blue colors may refer to 240° +/- 22°, i.e., a range from 218° to 262°.
- magenta colors may refer to 300° +/- 22°, i.e., a range from 278° to 322°.

In some embodiments, the additional colors/color groups may be defined. In some embodiments, the defined colors/color groups may be shifted compared to the values mentioned above.

In some embodiments, other color spaces or color models may be used, e.g., RGB, CIELAB, CIEXYZ, CMYK, HSL, TYB and/or CMY. The applicant is convinced that a person skilled in the art can convert the references to the HSV color space to any other color model.

Throughout the disclosure, a color scheme is understood as the complete palette of colors of the visual content and/or test image. In some embodiments, the color scheme may comprise only the most prominent and/or important colors or color groups. Furthermore, a color scheme may also be understood as a pre-defined color scheme and/or an expected color scheme. A pre-defined color scheme may be understood as a color scheme that typically is part of the visual content, and e.g., may defined by an software application used to view or analyze the visual content. An expected color scheme may be understood as a color scheme, that is expected due to the type or source of visual content. In some embodiments expected color scheme and pre-defined color scheme may overlap or be used synonymical.

In some embodiments, the actual color scheme of the visual content may differ from the expected color scheme of the visual content. In some embodiments, the color scheme of the visual content is pre-defined in dependence of the visual content. In some embodiments, the color scheme of the visual content is determined by the controller, e.g., by analyzing the visual content and/or applying a color space mapping on visual input data.

If not explicitly specified differently, a visual test might be understood to comprise any of the first visual test and modified visual test.

If not explicitly specified differently, a test image is a picture comprising at least one object and at least one background. The colors or color groups of the object and the background are preferably different from each other. In some embodiments, the boundaries between background and object may be fluent. For example, the colors may be very similar, although not in the same color group. If not explicitly specified differently, a test image might be any of the first test image and modified test image.

In some embodiments of the system, the visual test comprises at least one test image and an input prompt. The test image comprises at least one object and at least one background in different colors or color groups. The test image may comprise the full color scheme of the visual content or at least partially the color scheme of the visual content. In some embodiments, the test image may comprise a pre-defined color scheme, wherein the pre-defined color scheme is in dependence on the expected color scheme of the visual content. In some embodiments, the test image comprises at least two colors or color groups, wherein the two colors or color groups are the colors or color groups that need to be distinguishable by the user.

In some embodiments of the system, the test image may comprise a plurality of objects, wherein at least one of the plurality of objects needs to be correctly identified by the user. In some embodiments, the plurality of objects may be divided into correct objects and false objects, wherein the test image comprises at least one correct object and may comprise a plurality of false objects. Individual objects of the plurality of objects may be colored independently of each other in different colors or color groups. In some embodiments, at least two objects of the plurality of objects may be colored in colors from the same color group or in the same color. In some embodiments, the test image comprises a plurality of correct objects and the user needs to correctly identify all correct objects. The correct object may be colored in colors from the same color groups or are colored in the same color.

In some embodiments of the system, the input prompt of the visual test may comprise information about how to correctly identify the object in the test image. In some embodiments, the input prompt comprises information about to identify all correct objects in the test image. In some embodiments of the systems, the input prompt comprises an interactive section for the user to enter information about the identified object via an input device.

In some embodiments of the system, the test image may comprise interactive sections. In some embodiments, the object or the plurality of objects may be selectable by the user via an input device. In some embodiments, the input prompt asks the user to select all correct objects in the test image. This may be done by entering identifiers of the correct objects and/or by selecting the objects directly in the test image. In some embodiments, the identifier of the objects may be the objects itself. For example, an object may be a number or letter or a sequence of numbers and/or letters, then the input prompt may instruct the user to enter the number or letter or sequence of numbers and/or letters.

In some embodiments, the visual test may comprise a plurality of test images, wherein the input prompt instructs the user to select the test image in which he or she can identify the object comparatively better. The user may enter an identifier, e.g., a number or letter connected to the test image, and/or select the test image directly, e.g., by clicking on it, navigating a pointer onto the test image, and/or touch the respective area if the output device is combined with an input device, e.g., a touchscreen.

In some embodiments, the system is configured to save the outcome of the visual test together or linked to the visual content. This can be used to document that the user proved his ability to analyze the visual content and/or that the used hardware, e.g., displaying device, was qualified for such an analysis. For example, if the visual test was completed successfully, respective data may be stored linked to or with the visual content. Optionally, if the initial visual test was not successfully completed but a modified visual test, respective data regarding the modifications to the visual test and/or visual content may be stored with the visual content and/or linked to the visual content. In some embodiments, the modified visual content may also be saved.

An input device can be any device that allows a user to enter information to the system, including, but not limited to, keyboard, mouse, touch sensitive surface, a camera to register gestures, voice control, motions sensors capable of reacting to user gestures or tapping.

In a more specific example, the visual content may relate to diagnostics in the dental field. This may comprise images, videos, and other recordings captured by intraoral cameras, intraoral 3D scanners, x-ray techniques, and/or dental MRI. Recordings of intraoral cameras and/or intraoral 3D cameras may comprise recordings with diagnostic features, e.g., for caries detection by fluorescence and/or NIR. Additionally, or alternatively, the recordings may comprise further or other diagnostic features like lesions, inflammations, plaque, calculus, etc. Intraoral recordings may also feature color recordings, e.g., for shade detection or tooth color determination. Sometimes, these color images also serve for determination of the gingiva in intraoral scans. Recordings from fluorescence caries detection usually feature red colors and green colors as well as greyscale colors (including black and white). For the analysis of recordings from fluorescence caries detection, the user needs distinguish between red and green colored areas. The respective visual test thus should at least comprise red and green colors. If the first visual test is failed, red and/or green may be replaced by a different color for a modified test. If the modified visual test is then completed correctly, the respective color (red and/or green) that was replaced compared to the first visual test, may then also be replaced in the visual content by the same color. E.g., if red colors were replaced by blue colors for the modified visual test, red colors from the visual content may be replaced by blue colors to generate a modified visual content in which the user proved to be able to distinguish between the colors (e.g., blue colors and green colors).

To tell gingiva apart from actual teeth, the user needs to distinguish between white-yellow colors from a broad range of red colors. Thus, the visual test may comprise at least white-yellow colors and red colors. Optionally, the red colors may be predefined to lie close to the white-yellow colors. A series of test images may be generated to find out if a color may be replaced and/or the shading/saturation/brightness/contrast of the visual content may be modified, e.g., to generate a better separation between teeth and gingiva.

In most x-ray, NIR, and MRI recordings, the main colors are from the greyscale range (including black and white; sometimes referred to as monochromatic). Thus, the visual test may comprise test images in which fine differences between shades within greyscale need to be distinguished. Optionally, a series of test images may be used to find comparatively better settings of contrast and/or brightness of the visual content.

Sometimes, a user may review a series of recordings of the same kind, e.g., with the same colors/color groups to be distinguished, in a row or over a certain timespan. Eventually, the ambient lightning conditions in vicinity of the output device may change during this time. The system may optionally comprise a sensor and/or camera to sense a changing lightning condition. If e.g., a certain threshold of a value representing the lightning conditions is reached, the controller may generate a new visual test to test if the user is still able to distinguish the respective colors. Optionally, the visual test may comprise a plurality of test images of which the user is instructed to select the one in which the user can identify the object comparatively better. The settings (e.g., saturation, brightness, contrast, colors/color groups/color scheme) may then be applied to the visual content to eventually enhance the ability of the user to distinguish the respective colors. Alternatively, the test image of the visual test may comprise a plurality of objects and the user is instructed to select the object he can identify comparatively better.

Also, after some time has elapsed, the user's vision may be fatigued and the user's ability to distinguish certain colors may be deteriorated. Optionally, the controller may be configured to generate a new visual test after a certain amount of time to repeatedly test the user's vision.

Examples for visual content may be, but are not limited to, e.g.,
- medical recordings, images, or videos, such as
   ∘ 3D intraoral scans, CT, CBCT, NIR, MRI, fluorescence, 2D images,
   ∘ Oral recordings,
   ∘ Recordings of teeth and/or dental arches,
   ∘ Mammography recordings,
   ∘ Endoscopy and Laparoscopy recordings,
   ∘ Ultrasound data or recordings,
   ∘ X-Ray Recordings,
- Industrial and environmental recordings, images, or videos, such as
   ∘ Metrology system displays,
   ∘ Automatic inspection systems,
   ∘ Monitoring video data (visible spectrum, infrared etc.) for critical visual infrastructure/process monitoring (power plants, chemical industry, etc.),
- Military recordings, images, or videos, such as
   ∘ Drone control and surveillance,
   ∘ Night vision and infrared data/recordings,
   ∘ Radar and sonar,
   ∘ Laser targeting systems,
   ∘ Thermal vision,
   ∘ Satellite images.

### BRIEF DESCRIPTION OF THE DRAWINGS

In the following description, the present invention will be explained in more detail with reference to the exemplary embodiments and with reference to the drawings, wherein
Fig. 1 shows an exemplary embodiment of a system for displaying visual content;
Fig. 2 shows an exemplary embodiment of a visual content and respective visual test;
Fig. 3 shows an exemplary embodiment of a visual test;
Fig. 4 shows an exemplary embodiment of a visual test;
Fig. 5 shows an exemplary embodiment of a system including a sensor and imaging device;
Fig. 6 shows an exemplary embodiment of a system including an imaging device for aerial surveillance;
Fig. 7 shows an exemplary embodiment of a system featuring multiple output devices;
Fig. 8 shows a schematical depiction of connection between user settings and output devices according to an embodiment;
Fig. 9 shows an exemplary embodiment of a visual test with tooth shade chart;
Fig. 10 shows a block diagram according to an embodiment of a method for displaying visual content.

### DETAILED DESCRIPTION OF THE EMBODIMENTS

To be noted is, that throughout the description, the visual test will be referred to with the number 112, as the term "visual test" is used in its general meaning, i.e., regardless of if it is the first visual test or any modified visual test. If no adjective (first or modified) is used together with "visual test" both meanings, i.e., first and/or modified visual test, may apply.

The shown embodiments, especially the examples for the visual content, are purely of exemplary nature and are not limiting the scope of the invention.

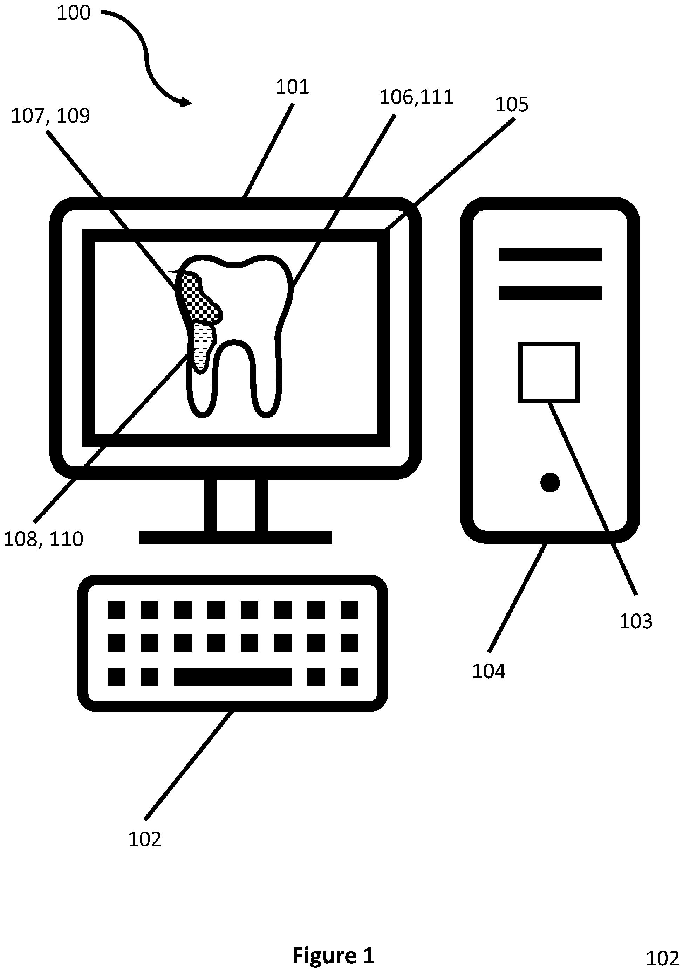

An exemplary embodiment of the system 100 for displaying visual content 105 is schematically shown in Fig. 1. The system comprises an output device 101, an input device 102, and a controller 103 which exemplarily is embedded in a computing device 104. Optionally or alternatively, the controller 103 may be embedded in any cloud-connected device, such as a server or an edge device, i.e., a local server. In some embodiments, the controller 103 may be embedded in a smartphone and/or tablet computing device. The functions of the controller 103 described herein may optionally be distributed to several different controllers on individual devices which optionally are in communication with each other. It is to understand, that a person skilled in the art knows how to distribute particular operations to controllers on different devices.

The controller 103 may further comprise at least one processor configured to analyze and/or process data regarding the visual content 105.

The output device 101, the input device 102, and the controller 103 (or computing device 104, respectively) are communicatively connected to each other. The connection maybe wired or wireless or a mixture of wired and wireless. In some embodiments, the controller 103 may be located on or be a part of a webserver (e.g., as a cloud-computing service).

The output device 101 may be any device capable of displaying visual content 105. For example, but not limited to, an output device 101 may be any kind of display or screen. For example, the output device 101 may be, but not limited to, a computing device screen, a tablet computing device screen, a smartphone display, and/or a projector.

The input device 102 may be any device capable of receiving input from a user. For example, but not limited to, an input device 102 may be a keyboard, a computing device mouse, a tracking ball, any pointing device, and/or any touch-sensitive surface. In some embodiments of the system, the input device 102 is combined with the output device 101, e.g., as a touchscreen.

The data of the visual content 105 may be stored locally on a computing device and/or a local server/storage device (e.g., edge device). Alternatively, or optionally, the data of the visual content 105 may be stored on a web server or in the cloud. In some embodiments, the visual content 105 is accessed via a cloud application or a web application.

In the exemplary embodiment of Fig. 1, the subject 106 of the visual content 105 is a medical recording, more specific, a recording of a tooth. It is to understand, that the visual content 105 alternatively may comprise any kind of recordings and is not limited by the examples shown herein. The recordings may be images and/or videos. In other embodiments, the visual content 105 may feature any medical content and is not limited to dental recordings. The visual content 105 comprises features, among these features are colors, color groups and a color scheme. In some embodiments, the features of the visual content 105 further comprise values for brightness, contrast, and/or saturation. In some embodiments, a pre-defined color scheme may be attributed to the visual content 105 in dependence of the expected color scheme of the visual content 105. For example, for visual content 105 featuring medical images for dental caries detection via fluorescence, the visual content 105 may be expected to have a color scheme mainly comprising red colors, green colors, and grey colors (i.e., greyscale, including black and white). Consequently, a pre-defined color scheme for the visual content 105 may as well mainly or exclusively comprise red colors, green colors, and, optionally, grey colors.

The visual content 105 may comprise colors and/or color groups that the user must be able to distinguish to analyze the visual content 105. The ability to distinguish the colors and/or color groups may depend on the vision of the user, the ambient lightning conditions, the output device 101, and/or further aspects that may influence the vision of the user.

The subject 106 may feature a characteristic 107 and a characteristic 108. Characteristic 107 is colored in a first color/color group 109. The characteristic 108 is colored in a second color/color group 110. The rest of the tooth, which does not feature any extra characteristic is colored in a third color/color group 111. In the exemplary visual content 105, the user may have to be able to at least distinguish color/color group 109 and color/color group 110 from each other.

To ensure that the user can distinguish color 109 from color 110 on the output device 101, the controller 103 is configured to generate at least one visual test 112 which comprises features of the visual content 105, e.g., colors, color groups, the color scheme of the visual content 105. Optionally, the visual test 112 may further comprise features of the visual content 105 like brightness, contrast, and/or saturation.

In some embodiments, the visual test 112 comprises expected features of the visual content 105. Expected features may be, but not limited to, a pre-defined color scheme and/or an expected color scheme. The expected features may be based on the type of visual content 105. In some embodiments, the expected features are based on software application to analyze the visual content.

With respect to the visual content 105 shown in Fig. 1, the color scheme for example comprises the colors/color groups 109, 110, 111. The visual test 112 generated by the controller 103 may at least feature the two colors/color groups that the user needs to be able to distinguish, namely the colors/color groups 109 and 110. In some embodiments, if the type of visual content 105 is known, e.g., a tooth with possible characteristics 107 and/or 108, the controller 103 may use a pre-defined color scheme to generate the visual test 112. For example, the pre-defined color scheme for the subject 106 of Fig. 1, the pre-defined color scheme consists of the colors/color groups 109, 110.

The controller 103 is configured to generate a visual test 112 using features of the visual content 105 to test if the user can distinguish certain colors of the visual content 105.

To generate the visual test 112, the controller 103 may be configured to analyze the visual content 105 to determine the color scheme or color groups that need to be distinguishable by the user. In some embodiments, the controller 103 may analyze the visual content to extract the most dominant colors/color groups.

For example, a fluorescence image of a tooth may be dominated by a third color/color group 111, e.g., gray, together with a first color/color group 109, e.g., green, and a second color/color group 110, e.g., red. The controller 103 thus may be configured to extract the colors/color groups 109, 110, 111 and use these colors/color groups to generate the visual test 112.

The visual test 112 comprises at least a first visual test 112 comprising at least one first test image 113. The test image 113 may feature at least one object 115. The visual test 112 may comprise features of the visual content 105, e.g., a color scheme, colors, color groups and/or brightness or contrast. If the user correctly identifies the object 115, the controller 103 is configured to allow displaying the visual content 105 on the output device 101.

If the user fails to correctly identify the object 115 in the test image 113, the controller 103 is configured to deny displaying visual content 105 on the output device 101.

In some embodiments, the controller 103 is configured to generate further visual tests, optionally with modified test images. These modified test images may comprise at least one change in the features of the visual content 105. For example, a color/color group may be substituted by a different color/color group. In a more specific, but not limiting, example, if the user cannot identify a green object 115 on a red background 114 in the test image 113, the controller 103 may generate a modified test image with a blue object 115 on a red background 114. If the user then correctly identifies the object 115, the controller 103 may be configured to modify the visual content 105 accordingly, for example by substituting green colors in visual content 105 by blue colors.

In some embodiments, the controller 103 is configured to generate a limited number of visual tests 112. If the user fails to correctly identify the object 115 in any of these visual tests 112, the controller 103 may be configured to block any further attempts of displaying the (modified) visual content 105.

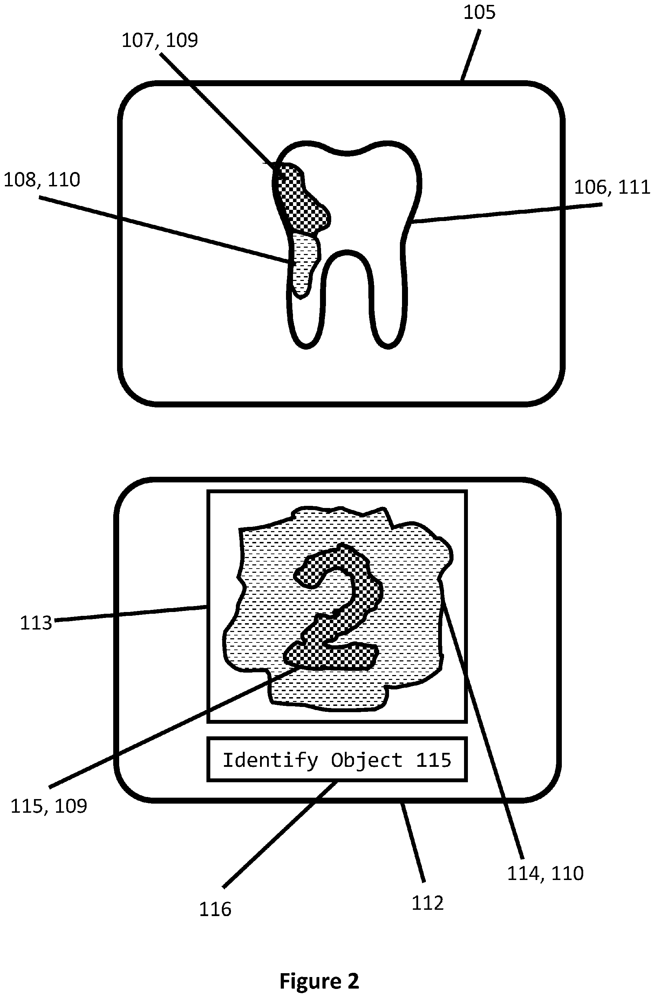

An exemplary embodiment of the visual content 105 and a related visual test 112 are schematically shown in Fig. 2.

As shown in Fig. 1, the visual content 105 exemplarily comprises a recording of a subject 106. The main body of the subject 106 is colored in color/color group 111. The subject 106 further comprises two characteristics 107, 108. The characteristic 107 is colored in color/color group 109 and the characteristic 108 is colored in color/color group 110. The characteristics 107, 108 of the subject 106 may be of particular interest to the user and thus need to be distinguishable for the user.

To ensure that the user can distinguish color 109 from color 110 on the output device 101, the controller 103 is configured to generate at least one visual test 112 which comprises features of the visual content 105, e.g., colors, color groups, the color scheme of the visual content 105.

The exemplary visual test 112 comprises a test image 113 and an input prompt 116. The test image 113 is generated by the controller 103 using features of the visual content 105. The test image 113 is generated in a way that the user may prove that he/she can distinguish the colors that need to be distinguished to analyze the visual content 105. The features of the visual content 105 used to generate the visual test 112, more specific the test image 113, exemplarily comprise the colors/color groups 109, 110. The exemplary test image 113 includes an object 115 colored in color/color group 109 and a background 114 colored in color/color group 110. The object 115 is embedded in or on the background 114. The object 115 of the test image 113 in Fig. 2 exemplarily is a number. In alternative or further embodiments, the object 115 may as well be a letter, a sequence of letters and/or numbers, and/or any shape. Optionally, the object 115 of the test image may be formed by a plurality of shapes. For example, the number shown in test image 113 of Fig. 2 may as well be composed of a plurality of circles and/or other shapes that are arranged in a way to form the respective number. The same applies to letters, sequences, and other shapes.

Optionally, the object 115 may comprise a plurality of colors and/or shades of the same color. Preferably, if the object 115 comprises a plurality of colors, the colors are of the same color group and are different from the color/color group of the background 114.

Fig. 2 shows that the test image 113 of the visual test 112 is generated using features of the visual content 105. The features of the visual content 104 used to generate the test image 113 exemplarily are the colors 109, 110 of the characteristics 107, 108 of the tooth 106.

In some embodiments the controller 103 may be configured to analyze the visual content 105 and extract the colors 109, 110, 111 that are found in the visual content 105 and at least partially use the extracted colors to generate the test image 113.

In some embodiments, the controller 103 may have access to pre-defined and/or expected color schemes that are optionally linked to specific visual content and/or visual tests. The pre-defined color schemes may comprise the colors that are expected to be found in the visual content 105. In some embodiments, the pre-defined color schemes may comprise the colors that need to be distinguishable to be able to analyze the visual content 105. For example, the visual content 105 of Fig. 2 comprises the colors 109, 110 and 111, whereas to analyze the visual content 105, the user must be able to distinguish color 109 from color 110. Accordingly, the pre-defined color scheme used to generate test image 113 may comprise at least the colors 109 and 110. Optionally, the controller 103 may be configured to extract the colors from the visual content 105 to generate a color scheme of visual content 105. The controller 103 may further be configured to compare the color scheme of visual content 105 to pre-defined color schemes and to use the color scheme that matches the color scheme of visual content 105 the closest to generate test image 113.

In some embodiments, the pre-defined color schemes only comprise colors that need to be distinguishable to analyze the visual content 105. For example, a pre-defined color scheme may consist of the colors 109 and 110 and the controller 103 may have found, that this pre-defined color scheme is closest to the color scheme of visual content 105, although the pre-defined color scheme does not feature the color 111. The controller 103 may then be configured to use the color scheme consisting of colors 109 and 110 to generate the test image 113.

In some embodiments, the controller 103 is configured to have access to the type of visual content 105 to be displayed. For example, the visual content 105 may be fluorescent recordings of a tooth 106 for caries detection. The information about the type of visual content 105 may for example contain information about the expected colors in the visual content 105. Optionally, the information about the type of visual content 105 may also include the colors that need to be distinguishable to analyze the visual content 105. Referring to Fig. 2, the information about the type of visual content 105 may contain the information, that the user needs to be able to distinguish between the color 109 of characteristic 107 and color 110 of characteristic 108. The controller 103 may be configured to use this information to generate the test image 113 of the visual test 112.

In some embodiments, the visual test 112 features an input prompt 116. The input prompt 116 may comprise instructions on how to identify the object 115 in the test image 113.

In some embodiments, the input prompt 116 may ask the user to click or point on the object 115 in the test image 113. The controller 103 may, for example, register if the correct area of the object 115 was selected.

In some embodiments, the input prompt 116 may ask the user to enter an identifier, e.g., a number, letter and/or alphanumeric sequence, of the object 115. In some embodiments, the object 115 itself can be the identifier. With respect to Fig. 2, the input prompt 116 may ask the user to enter the number of the object 115, in this exemplary embodiment the number "2". Accordingly, the input prompt 116 may comprise an area to input the identifier of the object 115.

When the user identified an object in the test image 113, regardless of if he/she identified the correct object, the user may use the input device 102 to enter information about the identified object. The controller 103 is configured to analyze the entered information to determine if the user correctly identified the object and/or identified the correct object(s) (in case the test image 113 comprises a plurality of objects). If the controller 103 determines that the user correctly identified the object or identified the correct object(s), the controller 103 allows displaying the visual content 105 via the output device 101. If the controller 103 determines that the identification of the object and/or objects was failed, the controller 103 is configured to deny displaying the visual content 105 on the output device 101.

Optionally or alternatively, the controller 103 may generate at least one modified visual test 112 if the first visual test 112 is failed by the user, i.e., the user did not correctly identify the object. The modified visual test 112 may differ from the first visual test 112 in at least one selected from the object, the background, at least one color and/or color group, and/or the color scheme. If the user correctly finishes the modified visual test 112, i.e., correctly identifies the object and/or all correct objects, the controller 103 is configured to modify the visual content 105 in dependency on the modifications of the visual test 112 to generate a modified visual content. For example, if the visual test 112 was modified in its color scheme, the original color scheme of the visual content may be replaced by the used color scheme in the modified visual test 112 to generate a modified visual content.

Optionally or alternatively, the controller 103 may generate choice options after a visual test 112 is failed. These choice options may comprise at least two options selected from the following:
- generating a subsequent modified visual test 112, wherein an object of the test image is modified, and wherein the color scheme, and brightness, and contrast of the preceding verification task are repeated. This option lets the user take the first visual test 112 again, in case he/she made a careless mistake, e.g., a typo when entering the object and/or identifier of the object, but in general can distinguish the colors and/or color groups from the test image. If the user correctly finishes this modified visual test 112, i.e., without modifications to the colors and/or color groups, the controller 103 may not generate a modified visual content but allow displaying the original visual content.
- generating a subsequent modified visual test 112, wherein the object in the test image as well as the color scheme and/or single color groups are modified compared to the preceding visual test 112. The user may choose this option, if he indeed was not able to distinguish the colors and/or color groups in the test image. If the user chose this option and correctly finishes the modified visual test 112, the controller 103 may generate modified visual content 105 in dependency on the modifications of the visual test 112 and allow displaying the modified visual content 105 on the output device 101.
- cancel, wherein the controller is configured to deny displaying the visual content 105 and/or not to generate modified visual content 105.

Further, the controller 103 may optionally generate choice options leading to a modification in contrast, brightness, and/or saturation of the test image and thus, if the user correctly finishes the modified visual test 112, may apply these modifications to the visual content 105 to generate the modified visual content 105.

If the user is not able to distinguish the colors and/or color groups in the test image 113, thus fails to identify the object and/or objects, the controller 103 is configured to generate a modified test image 113. Thes modifications of the test image 113 may comprise at least one selected from the following: the object, at least one color and/or color group, the color scheme, brightness, contrast, and/or saturation. In some embodiments, at least the object is changed. If the object is a sequence of numbers and/or letters, the arrangement and/or the numbers/letters may change such that the user does not see the same sequence twice in a row.

In some embodiments, when the user fails to identify the object 115 in the first test image 113, the controller 103 generates a modified test image 113 by modifying at least one selected from a color, a color group and/or the color scheme. The controller 103 may change the color and/or color group of the object 115 and/or of the background 110 to generate a modified test image 113. At least one color and/or color group that is changed is different from the colors and/or color groups in the preceding test image 113. In some embodiments, no color of the preceding test image 113 may be used again for the next modified test image 113. For example, if the first test image 113 comprises the colors red and green, the modified test image may comprise neither red, nor green.

In some embodiment, the controller 103 is configured to generate a modified visual content in dependency on the modifications of the test image 113 from the modified visual test 112. For example, the controller 103 may replace the color of the visual content 105 with the same color that was used to replace a color in the test image 113. In a more specific example, if red colors were replaced by blue colors in the test image 113 to generate a modified test image 113 and the user then correctly identifies the object 115, the controller may be configured to replace red colors by blue colors in the visual content 105 as well.

In some embodiments, the controller 103 may analyze the visual content 105 to identify the whole color palette of the visual content 105 to avoid replacing a color with a color that is already present in the visual content 105. For example, if the visual content 105 already comprises blue colors, the controller 103 may try to avoid replacing any other color with blue colors.

In some embodiments, the controller 103 may use machine learning algorithms or artificial intelligence or be linked or in communication with respective systems. These algorithms or artificial intelligence may be trained with previously recorded visual content 103 and may be configured to generate visual tests 112 that are similar or close to the visual content 103. In some embodiments, the controller 103 may thus generate a visual test 112 featuring test images 113 with, e.g., barely distinguishable objects 115. This kind of visual test 112 may additionally test the user's ability to identify even marginal characteristics 107, 108 in the visual content.

In some embodiments, the test image 113 may comprise a sequence of numbers, letter or a mixture of numbers and letters Optionally, the sequence of the object 115 may partially be colored in a first color or color group and partially in a color or color group different from the first color or color groups. For example, some of the numbers and/or letters may be colored differently than the other numbers and/or letters of the sequence. Optionally, every number and/or letter of the sequence may be colored individually. Optionally, if the numbers and/or letters of the sequence are colored in a plurality of colors or color groups, the background may be colored in a neutral color, e.g., white, black, and/or greyscale. In some embodiments, the test image 113 may comprise a plurality of colors or color groups, especially if the user needs to be able to clearly distinguish more than two colors and/or color groups. The input prompt 116 may then instruct the user to enter all numbers and/or letters of a specific color or color group. In some embodiments, the input prompt 116 may instruct the user to enter the numbers and/or colors of the sequence separated by their color and/or color group, e.g., the user needs to enter the numbers/letters of a first color/color group separately from the numbers/letters of a different color/color group.

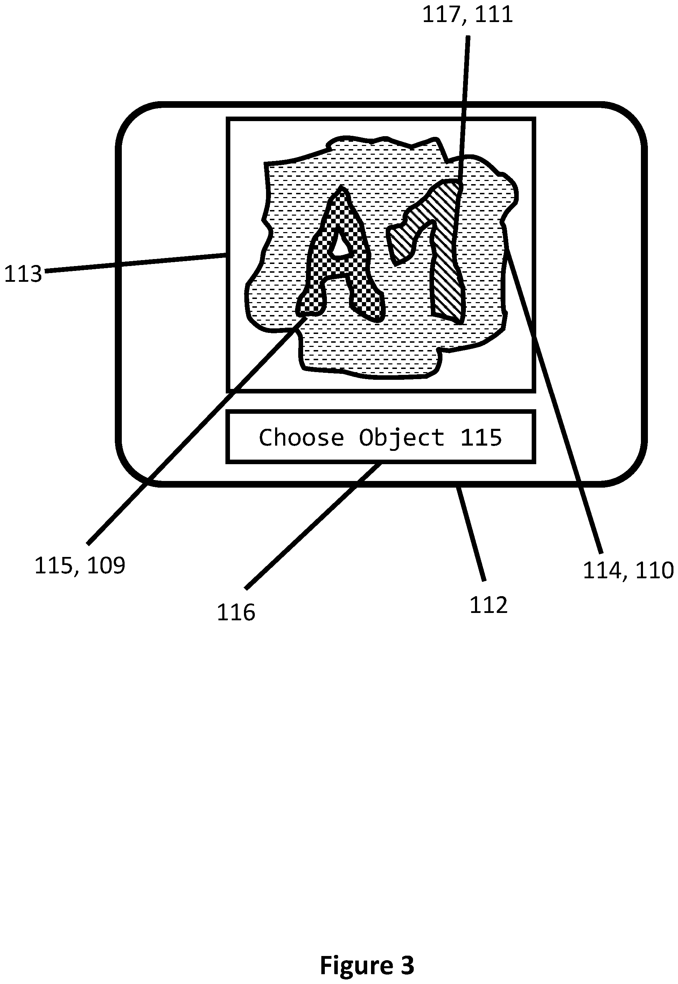

In some embodiments, the test image 113 may comprise a plurality of objects 115, 117 of which at least two objects 115, 117 are colored in different colors and/or color groups 109, 111 as shown in Figure 3. Optionally, the colors/color groups are those colors/color groups that the user needs to be able to distinguish as described above.

Exemplarily, the input prompt 116 asks the user to identify the object 115 in test image 113. For example, the input prompt may ask the user to choose the object of a certain color, e.g., the first color 109. In other embodiments, the test image 113 may comprise a plurality of objects 115 that need to be correctly identified and objects 117 that should not be selected or entered. The input prompt 116 may then ask the user to select or enter all objects 115 that are specified, e.g., by the color/color group.

In some embodiments, the visual test 112 may comprise multiple steps. For example, the visual test 112 may be aimed to prove that the user can correctly distinguish a first color 109 from a second color 110; a third color 111 from a second color 110; and a first color 109 from a third color 111. The input prompt 116 may first instruct the user to select the object colored in a first color 109 (object 115). If the user correctly identified object 115 as the object colored in the first color 109, the input prompt 116 may then, in subsequent step, ask the user to identify the object colored in the third color 111 (object 117).

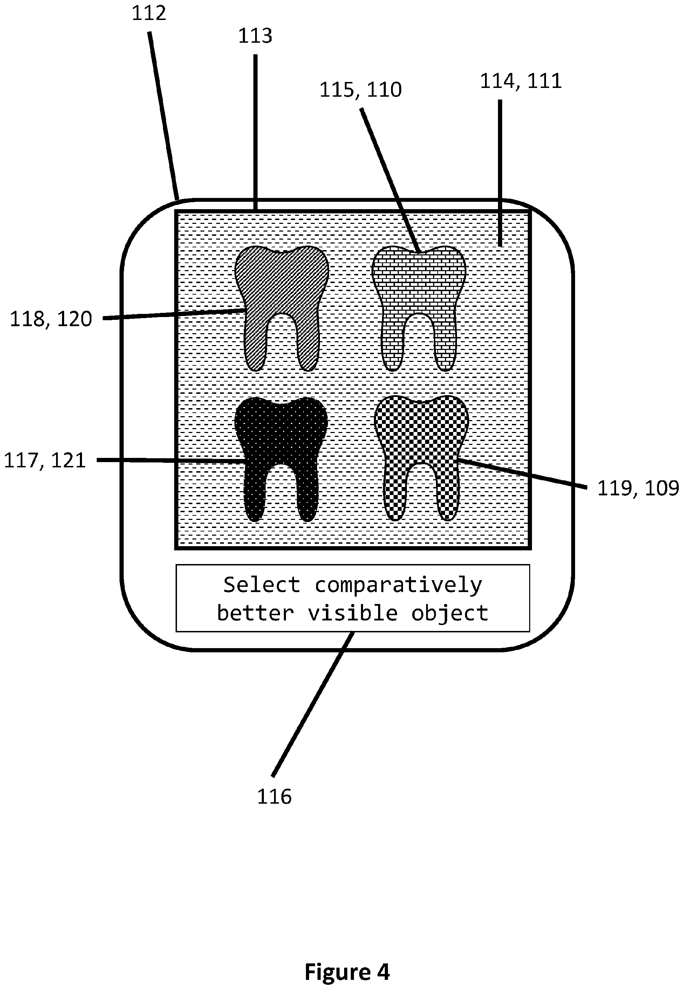

A further exemplary embodiment of a visual test 112 is schematically shown in Fig. 4. The test image 113 of this visual test 112 features four objects 115, 117, 118, 119 embedded on a background 114. It is to understand, that the number of objects in the test image 113 may vary and was randomly chosen to be four for exemplarily reasons. In some embodiments, the controller 103 may have information about the size and resolution of the output device 101 and thus may adapt the number of objects in the test image 113. For example, if the output device 101 is a large computing device display, the number of objects may be higher than for a small smartphone display.

A visual test 112 with a test image 113 comprising multiple objects may for example be used especially when the analysis of the visual content 105 requires the user to distinguish colors with only marginal differences, e.g., when the color group or color is the same but a difference in shades needs to be seen, e.g., a lighter color must be distinguishable from a darker shade of the same color. Further, in some embodiments, a visual test 112 with multiple objects may be generated as a modified visual test when the first visual test was failed by the user.

The background 114 of the test image may comprise a color/color group 111. Optionally, the background may feature a gradient. The gradient can be a gradient in contrast, brightness, saturation, and/or shading of a single color. Alternatively, or additionally, the gradient may be a color gradient within a color group. The direction and intensity of the gradient may vary. Optionally, the background 114 may comprise a plurality of gradients, e.g., in color, contrast, brightness, shading and/or saturation. The plurality of gradients may have the same direction, be at least partially overlapping, run parallelly, or may run in any angle to each other eventually intersect with each other.

In some embodiments, the background 114 may feature discrete areas that are individually colored. Optionally, the discrete colors may overlap or fade into each other in a way, that no clear border between these areas is visible. Optionally, the discrete areas may be arranged in a way that in each of the areas an object is embedded. In some embodiments, the color of the respective area of the background is in dependency on the color of the embedded object. Optionally, the color of the respective discrete area of the background is a complementary color to the color of the embedded object.

Exemplarily, the test image 113 of Fig. 4 features four objects 115, 117, 118, 119. Object 115 is colored in color/color group 110, object 117 is colored in color/color group 121, object 118 is colored in color/color group 120, and object 119 is colored in color/color group 109. The colors/color groups 109, 110, 120, 121 may be independently from each other different colors and/or color groups. Optionally, two or more objects may feature colors from the same color group. In some embodiments, two or more objects may be the same color but in a different shading.

With respect to the optional discrete areas of the background 114 for each of the objects 115, 117, 118, 119, the objects and the respective discrete area of the background 114 may independently from each other vary in contrast and/or brightness, e.g., the contrast between object 115 and the background 114 may be different from the contrast between object 117 and background 114, while object 115 and 117 are colored in substantially the same colors.

The input prompt 116 may comprise instructions for the user how to correctly identify an object in the test image 113. For example, the input prompt 116 may instruct the user to select the object which he/she can identify comparatively better. The object will be identified correctly, if the user chooses any of the objects 115, 117, 118, 119 of test image 113. Optionally, the controller 103 may do a plausibility check on the input of the user. For example, if the user selects object 115, which is colored in a color 110 that may be very close to color 111 of the background 114 while object 117, which is colored in color 121, that may be very different to color 111 of background 114, the controller 103 may decide, that the choice is not plausible. If the controller 103 concludes that the choice is not plausible, the visual test 112 may considered to be failed, i.e., the object was not identified correctly. In some embodiments, a modified visual test 112 may then be generated, e.g., with modifications only in the shape/number/sequence/identifier of the object. In some embodiments, a notification may be generated giving the user information about the plausibility of his/her choice.

If the user selects the object which he can identify comparatively better, the controller 103 may allow displaying the visual content 105. If the color, color group, contrast and/or brightness of the selected object, optionally in combination with the background 114, differs from the respective features of the visual content 105, the controller 103 is configured to modify the visual content 105 with dependency on the differences or modifications made to reach the identified object selected by the user.

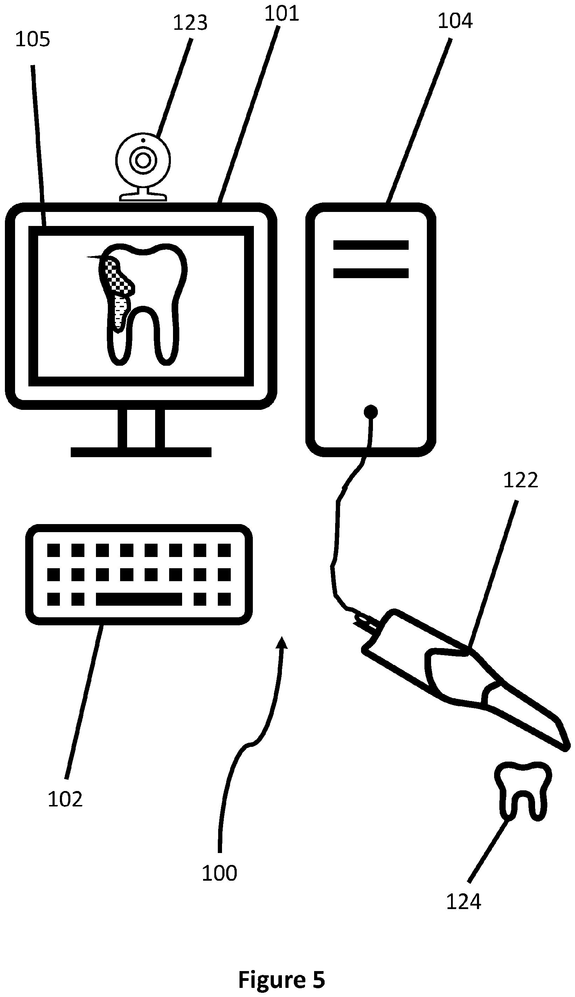

Another exemplary embodiment of the system 100 is schematically shown in Figure 5. Additionally, to the described features with respect to Figure 5, the exemplary embodiment may optionally also comprise features described with respect to any other disclosed embodiment.

The system 100 comprises a display as output device 101 to display the visual content 105 as well as the visual test 112 to verify if the visual content 105 can be displayed. The system 100 exemplarily features a keyboard as input device 102. The input device may additionally comprise a pointing device such as a computing device mouse, trackball or similar. In some embodiments, the input device 102 can comprise a touch sensitive surface such as a touchscreen combined with the display of the output device 101.

The system further features a computing device 104, exemplarily featuring the controller 103. In some embodiments, the controller 103 may be at least partially located on a web server or a cloud computing device or similar. The computing device 104 and/or the controller 103 are configured to process images, videos and/or data of a structure 124 recorded by the imaging device 122. Furthermore, the computing device 104 and/or controller 103 are optionally configured to analyze the recorded data, images, and/or videos at least for the purpose to generate a visual test 112 according to the recorded data, images, and/or videos. The recorded images, videos, and/or data can be further processed to be displayed as visual content 105 or directly be shown as visual content 105.

Here, the imaging device 122 exemplarily is an intraoral scanner, for example to record data, images, and/or videos of the dental arch, or single teeth (structure 124) of a user.

The system 100 may further comprise a sensor 123 to sense ambient lightning conditions. The ambient lightning conditions may be at least one of a brightness, color, light temperature. For example, the sensor 123 may sense a change in brightness due to a change from noon towards dusk, e.g., the brightness may decrease to due setting sun. Additionally, the sensor 123 may be configured to sense a change in light temperature, e.g., when an artificial light with a cold light temperature is switched on when the natural light with a warm light temperature is not sufficient or similar. In some embodiments, the sensor 123 alternatively or additionally may sense a change in the color of the ambient lightning conditions, e.g., when red colors from dusk or dawn are predominant and/or intensify and/or fade.

Upon sensed changes in ambient lightning conditions by the sensor 123, the controller 103 is configured to generate a new visual test 112 to consider the changed ambient lightning conditions. For example, when red colors become dominant during dusk, specific colors may be more difficult to distinguish by the user. Thus, the controller 103 generates a new visual test 112 to test, if the user still can distinguish these colors. The visual test 112 may be created and conducted the same way as described before and/or hereafter.

In another example, the ambient lightning conditions may get brighter, e.g., because an artificial light is switched on or stronger sunshine. The controller 103 may take such a change sensed by the sensor 123 into account by generating a new visual test 112, e.g., to test if the contrast and/or brightness of the output device 104 is still sufficient.

In some embodiments, the visual content 105 may be a live stream of video/images. The purpose of these images/videos may be known, such as a visual test 112 will be generated featuring colors or a color scheme to be expected from the live stream. Exemplarily, to display such a live stream, the visual test 112 is generated and conducted by the controller 103. If the user fails to correctly complete the visual test 112, displaying the live stream on the output device 101 may be denied.

In some embodiments, the visual content 105 comprises instructions, details, and/or images regarding a surgery and/or medical treatment, for example a dental surgery. Additionally, in some embodiments, the controller 103 may be operatively coupled and/or in communication with a medical instrument or an according instrument controller. The medical instrument may additionally or optionally be coupled to a dental chair or a medical instrument hub, which in turn is in communication or operatively coupled to the controller 103. If, in this embodiment, the visual test 112 is not completed correctly, the controller 103 can be configured to deny displaying the visual content 105 as well as deny the use of any medical instrument. This prevents the use of a medical instrument by a user that cannot certainly identify the areas that need treatment and may prevent unnecessarily damaging healthy areas.

In some embodiments, the controller 103 may generate a new visual test 112 after a time interval. The time interval may be predetermined, e.g., after a couple of hours, or randomly selected by the controller 103. The new visual test 112 after a time interval may consider, that the vision of the user can have worsened after a time interval, e.g., caused by fatigue.

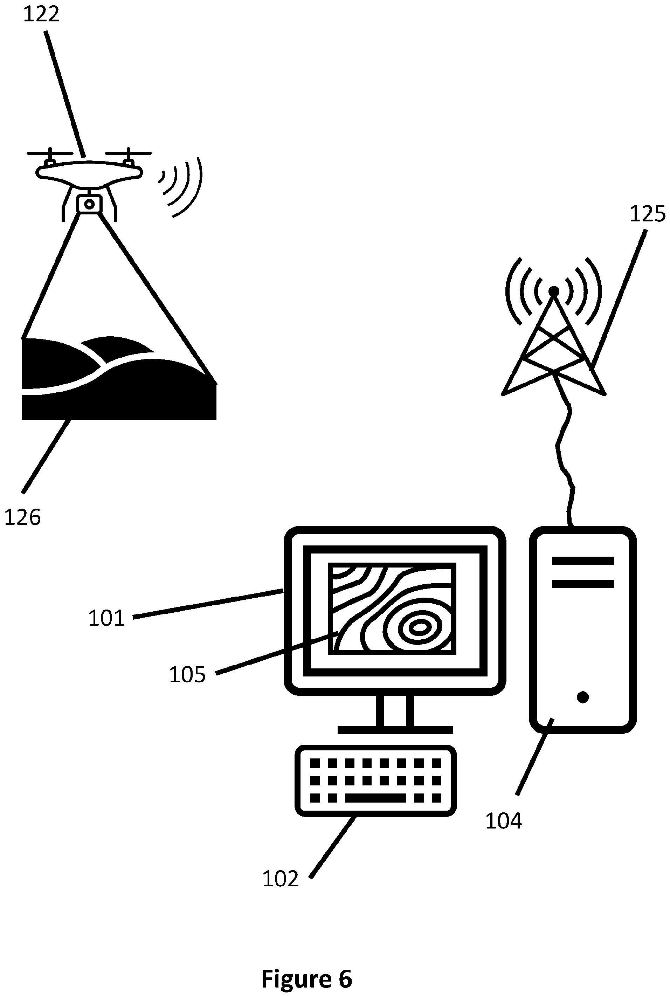

An exemplary embodiment of a non-medical use of system 100 is schematically shown in Figure 6. In the shown embodiment, the visual content 105 features videos, images, and/or data from an imaging device 122. In this embodiment, the imaging device 122 exemplarily is an aerial drone which records a landscape 126 and submits these recordings t an antenna 125. The visual content 105 thus may be a live stream of the landscape 126.

As described before, the system 100 comprises at least an output device 101, a computing device 104 at least partially comprising the controller 103, and an input device 102.

The visual content 105 of the landscape 126 may comprise military, scientific and/or security-relevant features. In some embodiments, the visual content 105 may be shown to identify forest fires, oil spillage, infrastructure, creatures (human and non-human), and similar.

The visual test 112 may be generated accordingly from a pre-defined color scheme with colors that need to be distinguishable in order to analyze the visual content 105. In some embodiments, the visual test 112 may be a series of test images 113 with different colors to distinguish as the recorded images, videos and/or data may comprise a variety of characteristics in different colors. For example, the visual content 105 may comprise a coastal area with green (vegetations), yellow/grey (sand, rocks), and/or blue (water) colors as well as hazards, e.g., red (fire), brown/black (oil spillage) colors.

In other embodiments, the visual content 105 may comprise spectral images, e.g., for scientific purposes or thermal vision.

In some embodiments, the imaging device 122 is communicatively connected to a computing device 104, wherein the computing device 104 is an edge device, local server, web server and/or cloud system. The connection may be wireless or wired via direct connection (e.g., edge device or local server in a local network) or via internet (e.g., to web server, cloud system, etc.). The output devices 101a, 101b, 101c, 101d may also be connected to the computing device 104 via local network and/or the internet.

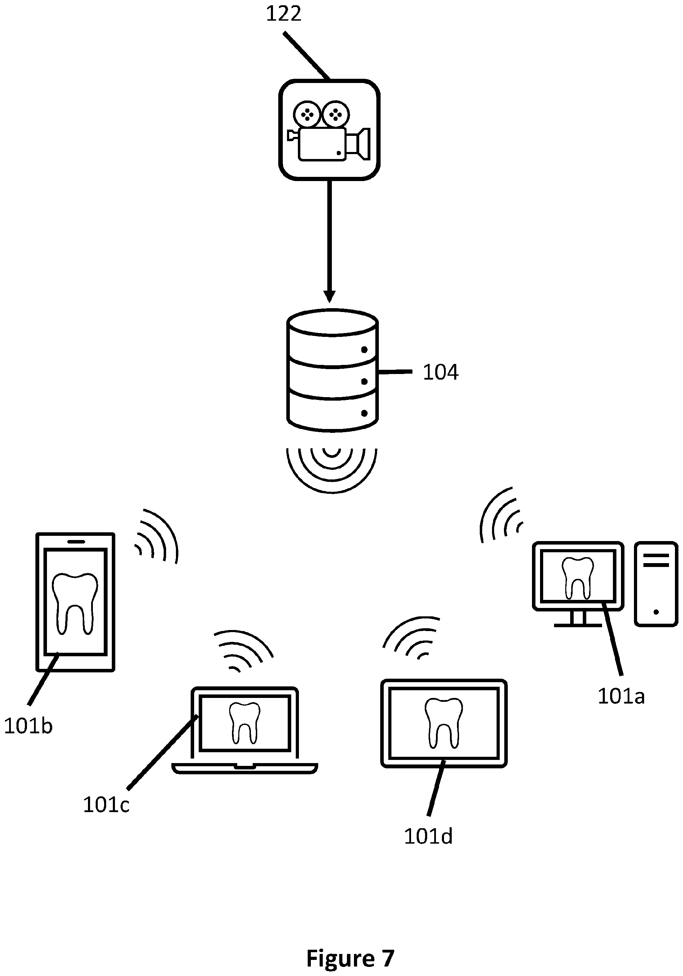

For the further description of figure 7, it is assumed, that the computing device 104 is a cloud system and the imaging device 122 as well as the output devices 101a, 101b, 101c, 101d are connected via an internet connection to the computing device 104. It is to understand that the described system with respect to figure 7 may work in the same fashion for different connections between computing device 104, imaging device 122 and output devices 101a, 101b, 101c, 101d and a person skilled in the art may easily derive changes necessary to enable different connection types. The described embodiments are of exemplary purpose and do not limit the scope of the invention as claimed.

A variety of output devices 101a, 101b, 101c, 101d is connected to the computing device 104 to display the visual content 105 recorded by the imaging device 122 or derived from these recordings. However, the various output devices 101a, 101b, 101c, 101d may comprise different display specifications which may result in different displaying properties, e.g., brightness, contrast, color. Characteristics of the visual content 105 that may be distinguishable by the user on one of the output devices 101a, 101b, 101c, 101d may on not be distinguishable on another one of the output devices 101a, 101b, 101c, 101d. Thus, it might be necessary to have a visual test 112 for each of output devices 101a, 101b, 101c, 101d before displaying the visual content 105.

For example, a user might analyze the visual content 105 on a first, exemplarily stationary, output device 101a and then continue analysis on a second, mobile output device 101b. A new visual test 112 may be created by the controller 103 after requesting access to the visual content 105 for output device 101b. Upon completion of visual test 112 on the second, mobile output device 101b, the visual content 112 eventually is modified according to the result of the visual test 112 on the second output device 101b.

Furthermore, a different user may also request displaying visual content 105 on a third output device 101c. Then again, a visual test 112 is generated by the controller 103 in order to verify that the user can distinguish the necessary characteristics and/or colors in the visual content 105.

In some embodiments, the controller 103 may be configured to store results and possible modifications to the visual content 105 for each user and each output device 101a, 101b, 101c, 101d. Optionally, the controller 103 may be configured to generate a visual test 112 with respect to the result of the last visual test 112 by a specific user on a specific output device 101a, 101b, 101c, 101d when new visual content 105 is requested to be displayed. Thus, the controller 103 can already take known specifics of the user (e.g., visual impairments) or the output device 101a, 101b, 101c, 101d into account and reduce the number of visual tests 112 or test images 113 to be completed before displaying the visual content 105.

In some embodiments, the controller 103 may save the results and possible modifications to visual content 105 as settings for the user that completed the visual test 112. The results and possible modifications may be associated to a specific user for a specific output device. Alternatively or additionally, the settings may also be associated to an output device 101 only, especially if the possible modifications do not comprise a change of colors, color groups or color scheme. For example, the controller 103 maybe configured to associate specific changes in brightness and contrast to a specific output device 101 and load these changes the next occasion when visual content 105 is to be displayed on that specific output device 101.

Optionally, this may be limited to cases where the visual content 105 to be displayed is of the same kind as when the original visual test 112 was completed and the according possible changes were saved.

In some embodiments, the controller 103 is configured to use the results of the visual test 112 to configure the output device 101 to have a comparable better display of visual content 105 as without this configuration.

In some embodiments of the system 100, it is mandatory for each user to complete a visual test 112 for every new output device 101 to display the visual content 105. The controller 103 may be configured to generate a visual test 112 when a new output device 101 is detected.

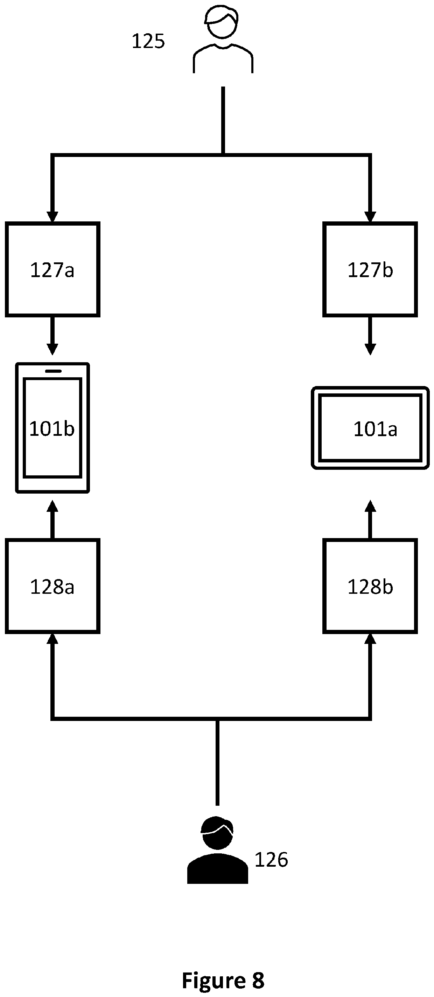

With respect to figures 7 and 8, the controller 103 may in some embodiments be configured to store multiple user settings 127a, 127b, 128a, 128b which are each associated with a specific user 129,130 and/or output device 101a, 101b, 101c, 101d. The user settings 127a, 127b, 128a, 128b may at least partially automatically derived from completion of the visual test 112. The user settings 127a, 127b, 128a, 128b may comprise information on at least one of color, color scheme, brightness, contrast.

In some embodiments, the controller 103 may be configured to automatically load the user settings 127a, 127b, 128a, 128b when the specific user 129, 130 uses one of the output devices 101a, 101b, 101c, 101d for displaying visual content 105. In some embodiments, a visual test 112 may be generated based on the saved user settings 127a, 127b, 128a, 128b to verify that the user settings 127a, 127b, 128a, 128b are still valid or need adjustment.

In some embodiments, the controller 103 may be configured to apply the user settings 127a, 127b, 128a, 128b and skip the visual test 112 if the ambient lightning conditions, e.g., sensed by a sensor 123, match the conditions when the former visual test 112, on which the user settings user settings 127a, 127b, 128a, 128b are based, was completed.

In some embodiments, the visual content 105 may comprise tooth color data or data about tooth shading. Usually, there is a discrepancy between the colors displayed on an output device 101, e.g., a display, and the actual color. For esthetic restorations of teeth, it may become necessary to be able to generate an impression of the final color of the restoration.

Usually, a tooth shade chart 129 is used and held against the teeth to identify the matching tooth shade 129a-j. Such a tooth shade chart 129 comprises multiple color samples resembling different tooth shades 129a-j. On some occasions, an image of the tooth shade can be recorded and displayed on an output device 101. To ensure that the displayed tooth shade is as close to the original, a visual test 112 may be generated including a series of test images 113 with different tooth shades 129a-j.

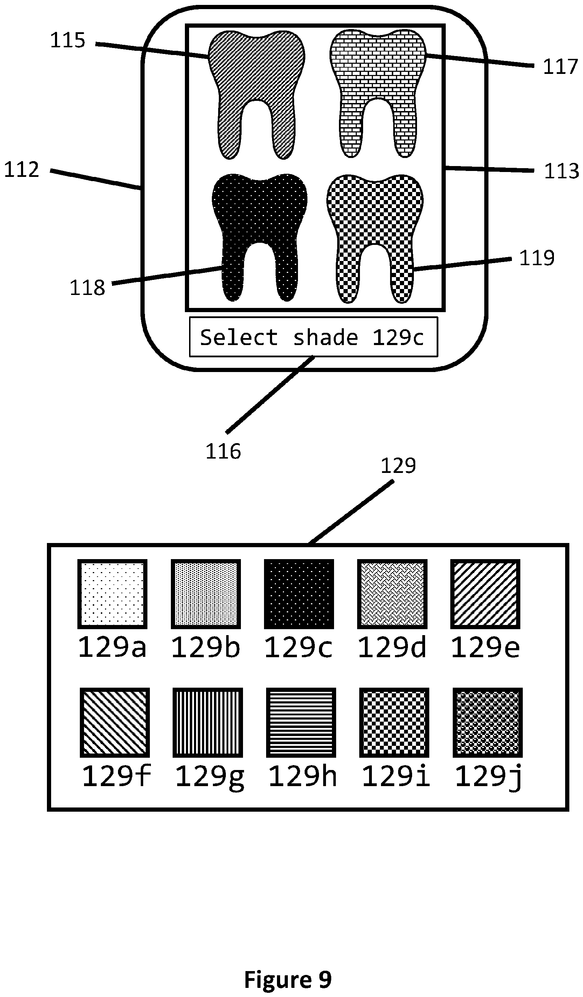

In some embodiments, the user is prompted to use the tooth shade chart 129 and compare the displayed objects 115, 117, 118, 119 colored in different tooth shades 129a-j to the tooth shade chart 129. The user might be prompted to select the object 115, 117, 118, 119 that matches the asked tooth shade the closest. For example, in figure 9 the input prompt 116 asks the user to select the object 115, 117, 118, 119 that matches tooth shade 129c the closest.

The controller 103 may be configured to adjust the colors of the output device 101 and/or the visual content 105 accordingly. For this adjustment, information on spectral or color data of each tooth shade 129a-j can be stored and matched with the originally recorded data. Upon completing the visual test 112, the controller 103 is configured to shift the colors in the visual content 105 for displaying on output device 101 according to the result from the visual test 112 while maintaining the shade information. Thus, each point of the visual content 105 may have a value of color to be displayed as well as information of the originally measured shade.

In some embodiments, the controller 103 may be configured to do a plausibility check on the user choices when prompted to select a tooth shade in the test image 113. For example, the controller 103 can be configured to compare the color of the selected object 115, 117, 118, 119 with an expected value or shade number and display a warning if a too great deviation is observed. For example, the visual test 112 may ask to identify an object with a dark yellow shade and the user selects an object with color values of a bright white shade, the controller 103 may be configured to interpret this selection to be not plausible and accordingly generate a warning indicating the deviation.

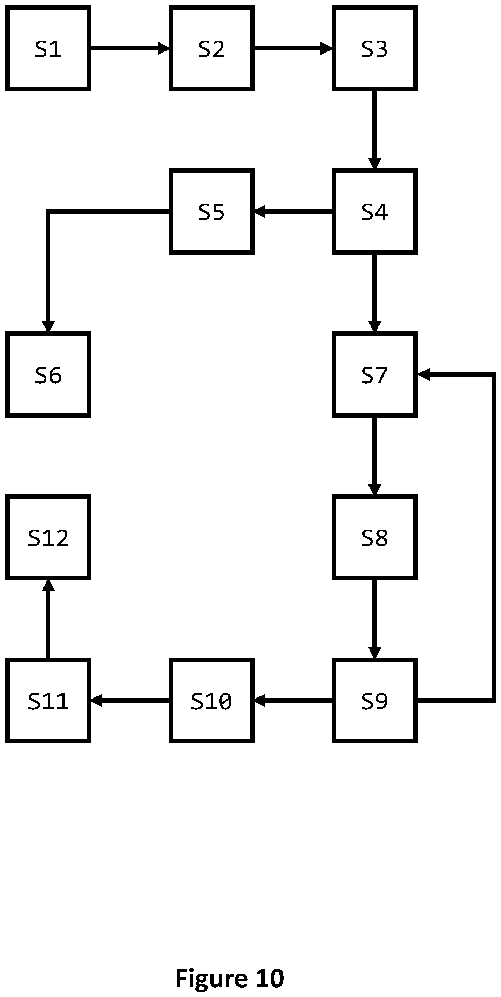

A flow chart visualizing the steps of an exemplary embodiment of the method for displaying visual content 105 is shown in figure 10.

In a first step S1, a user may request displaying visual content 105. The visual content 105 can be any image, video or other data that can be displayed via an output device 101. In some embodiments, the type of content of the visual content 105 is pre-defined and, for example, is bound to an application used for analyzing the visual content 105.

In a second step S2, a visual test 112 is generated using features of the visual content 105. The features may be color, color groups, a color scheme, brightness, contrast, etc. The visual test 112 may be generated by using features from a computing device-implemented analysis of the visual content 105, especially regarding the color and/or color scheme. In some embodiment, the visual test 112 is generated using pre-defined color schemes, e.g., based on the type of content of the visual content. In some embodiments, the generation of the visual test 112 may be assisted by an artificial intelligence or machine learning algorithms trained with previously recorded and analyzed visual content. The visual test 112 may contain an input prompt 116 indicating how to correctly complete the visual test 112. In some embodiments, the visual test 112 asks the user to correctly identify one of different objects 115, 117, 118, 119 from a test image 113.

In a third step S3, a user input is received by the system 100 via an input device 102. The user input contains information for identifying an object 115, 117, 118, 119 in the test image 113.

In a further step S4, the user input is analyzed if the object 115, 117, 118, 119 in the test 113 image was identified correctly. If the object was identified correctly, the method moves to step S5. If the object was not identified correctly, the method moves to step S7.

The method continues with step S5 if the identified object from the user input in step S3 was regarded as being correct. In step S5, the requested visual content 105 from step 1 is allowed to be displayed.

In the following step S6 the visual content 105 will be displayed on an output device 101. Step S6 is one possible end point of the method.

If step S4 results in the object being not identified correctly, the method continues with step S7 which comprises modifying the visual test 112. The modification of the visual test 112 may comprise a change of the brightness, contrast, particular colors and/or the color scheme used for test image 113. In some embodiments, the modifications may depend on the user input from step S3.

In a further step S8, another user input is received by the system 100 via an input device 102. The user input contains information for identifying an object 115, 117, 118, 119 in the modified test image 113.

In a further step S9, the user input from step S8 is analyzed if the object 115, 117, 118, 119 in the modified test 113 image was identified correctly. If the object was identified correctly, the method moves to step S10. Of the object was not identified correctly, the method may return to step S7, the modification of the visual test 112. In some embodiments, the method will stop by denying the display of visual content 105 after a certain number of iterations, i.e., modified visual tests 112. The maximal number of iterations can be any number and may be hard coded or set by a user.

In a further step S10, the visual content 105 is optionally modified based on the modifications of visual test 112 and/or test image 113 in step S7. For example, the color scheme, brightness, and/or contrast of the original visual content 105 may be shifted to color scheme, brightness, and/or contrast of the modified visual test 112. In a more detailed example, if a visual content 105 contains red and green colors and the user was not able to correctly identify a green colored object in the visual test of step S2, the green color maybe substituted with a blue color for the modified visual test 112 of step S7. If the user then correctly identifies the blue colored object, the green color of the original visual content 105 may be shifted to the blue color of modified visual test 112 from step S7.

In a further step S11, displaying of the modified visual content 105 will be allowed as the visual test 112 was completed correctly in step S8 and S9.

The method ends with step S12 by displaying the (modified) visual content 105 on output device 101.

## Claims

1. System for displaying visual content (105), comprising at least one output device (101), and at least one controller (103), and at least one input device (102), wherein
• the visual content (105) comprises features comprising a color scheme and/or at least one color group,
• the output device (101) is configured to display the visual content (105),
• the controller (103) is configured to generate a first visual test (112) comprising at least one first test image, and to allow displaying the visual content (105) on the output device (101) if a user correctly identifies an object (117, 118, 119) in the first test image, and to deny displaying the visual content (105) on the output device (101) if the user fails to correctly identify the object (117, 118, 119) in the first test image,
• the input device (102) is configured to receive input by the user to identify the object (117, 118, 119) in the first test image,
wherein the first test image (113) is generated using features and/or expected features of the visual content (105).

2. System according to claim 1, **characterized in that** the controller (103) is configured to generate a modified visual test (112) if the user fails to correctly identify the object (117, 118, 119) in the first visual test (112) and to allow displaying the visual content (105) or a modified visual content (105) on the output device (101) if the user correctly identifies an object (117, 118, 119) in the modified visual test (112), wherein the controller (103) is configured to generate further modified visual tests (112) until the user correctly identifies an object (117, 118, 119) in at least one of the further modified visual tests (112) or a maximum amount of failed modified visual tests (112) is reached.

3. System according to claim 1 or 2, **characterized in that** the controller (103) is configured to generate a modified visual content (105) by modifying the visual content (105) in dependency on the modified visual test (112) if the modified visual test (112) was completed correctly, and wherein the modifications of the visual content (105) are in dependency of the modifications of the visual test (112), and wherein the controller (103) is configured to allow displaying the modified visual content (105) on the output device (101) if the modified visual test (112) was completed correctly.

4. System according to any one of claims 1 to 3, **characterized in that** the modified visual test (112) comprises at least one modified test image (113) which differs from the test image (113) of the preceding visual test (112) in at least one or more from the following: the object (117, 118, 119), at least one color, at least one color group, the color scheme, brightness, and contrast.

5. System according to any one of claims 1 to 4, **characterized in that** first visual test (112) and/or the modified visual test (112) comprises a plurality of objects (117, 118, 119) that differ from each other in at least one selected from brightness, contrast, a color and/or a color group, wherein the controller (103) generates an input prompt instructing the user to select the object (117, 118, 119) which the user can identify comparatively better, and wherein the controller (103) is configured to apply the color and/or color group and/or brightness and/or contrast of the selected object (117, 118, 119) to the visual content (105).

6. System according to any one of claims 1 to 4, **characterized in that** first visual test (112) and/or the modified visual test (112) comprises a plurality of test images (113) that differ from each other in the object (117, 118, 119) and at least one selected from brightness, contrast, a color, a color group, and the color scheme, wherein the controller (103) generates an input prompt instructing the user to identify the object (117, 118, 119) of the test image (113) in which the user can identify the object (117, 118, 119) comparatively better, and wherein the controller (103) is configured to apply the color scheme and/or brightness and/or contrast of the selected test image (113) to the visual content (105).

7. System according to any one of claims 1 to 6, **characterized in that** the controller (103) is configured to generate the plurality of test images (113) and/or objects (117, 118, 119) in dependence on the completion of the first visual test (112).

8. System according to any one of claims 1 to 7, **characterized in that** the controller (103) is configured to generate choice options after a visual test (112) is failed, wherein the choice options comprise at least two options from the following:
(a) generating a subsequent modified visual test (112), wherein an object (117, 118, 119) of the test image (113) is modified, and wherein the color scheme, and brightness, and contrast of the preceding verification task are repeated, and
(b) generating a subsequent modified visual test (112), wherein the object (117, 118, 119) in the test image (113) as well as the color scheme are modified compared to the preceding visual test (112), and
(c) cancel, wherein the controller (103) is configured to deny displaying the visual content (105) and/or not to generate modified visual content (105).

9. System according to any one of claims 1 to 8, **characterized in that** the controller (103) is configured to generate, together with visual test (112) and/or after the visual test (112) is failed, a query to retrieve information from the user if and/or why the user is not able to correctly identify the object (117, 118, 119) in the test image, wherein the query comprises at least two choice options selected from contrast, brightness, color scheme, color, and color group, and wherein the controller (103) is configured to generate a modified visual test (112) based on the information retrieved from the user.

10. System according to any one of claims 1 to 9, **characterized in that** the controller (103) is configured to generate a new visual test (112) after a time interval and/or at specified moments and/or before every access to a different visual content (105) is requested by the user.

11. System according to any one of claims 1 to 10, **characterized in that** controller (103) is configured to request a validation before an output device (101) is used to display visual content (105) for the first time in the system, wherein the validation comprises at least one visual test (112) and the controller (103) is configured to allow displaying the visual content (105) and/or modified visual content (105) on the output device (101) only when the respective output device (101) is validated.

12. System according to any one of claims 1 to 11, **characterized in that** the system comprises a plurality of output devices (101), wherein each of the plurality of output devices (101) can be configured to display the visual content (105) and/or modified visual content (105), wherein each output device (101) is operated individually and needs to be validated before the first use in the system.

13. System according to any one of claims 1 to 12, **characterized in that** when at least two output devices (101) are used in the system, at least one output device (101) can be excluded from the mandatory validation, wherein the controller (103) is configured to allow displaying the visual content (105) and/or modified visual content (105) on the output device (101) that is not validated, and wherein the controller (103) is further configured to generate a notification on the output device (101) that is not validated, that an interpretation of the visual content (105) and/or modified visual content (105) on the output device (101) that is not validated is not allowed/safe.

14. System according to any one of claims 1 to 13, **characterized in that** the system comprises at least one data storage, wherein the controller (103) is configured to store user settings in the data storage, wherein the user settings are in dependency on the completion of the visual test (112) and/or the validation, and wherein the user settings comprise at least a color scheme, and wherein the user settings are automatically generated during the validation and/or visual test (112).

15. System according to any one of claims 1 to 14, **characterized in that** the user settings are linked to individual output devices (101), such that if a user uses a first output device (101), the user settings for the first output device (101) are loaded and then the user uses a different output device (101), the settings for the different output device (101) are loaded.

16. System according to any one of claims 1 to 15, **characterized in that** the system comprises at least one sensor and/or camera configured to sense at least partly the light conditions in vicinity of the output device (101), wherein the controller (103) is configured to generate a visual test (112) if the sensor and/or camera senses that the light conditions changed compared to the light conditions at a time when the preceding visual test (112) was generated.

17. System according to any one of claims 1 to 16, **characterized in that** the system is configured to analyze the colors and/or color scheme of the visual content (105), and the controller (103) is configured to generate the test image (113) and/or plurality of test images (113) in dependence of the analysis.

18. System according to any one of claims 1 to 17, **characterized in that** the controller (103) is configured to generate the test image (113) and/or plurality of test images (113) in dependence of a pre-defined setting, wherein in the pre-defined setting comprises a color scheme.

19. Computer-implemented method for displaying visual content (105), wherein the visual content (105) comprises features comprising a color scheme and/or at least one color group, the method comprising the steps of
(a) generating a first visual test (112), comprising a first test image (113),
(b) receiving input from a user identifying an object (117, 118, 119) in the first test image (113),
(c) allowing displaying the visual content (105) on an output device (101) if the user correctly identifies the object (117, 118, 119) in the first test image (113), and denying displaying the visual content (105) on the output device (101) if the user fails to correctly identify the object (117, 118, 119) in the first test image (113),
wherein the first test image (113) is generated using features and/or expected features of the visual content (105).
